(19) **Europäisches Patentamt**
**European Patent Office**
**Office européen des brevets**

(11) **EP 2 133 808 A1**

(12) **EUROPEAN PATENT APPLICATION**

(43) Date of publication:
**16.12.2009 Bulletin 2009/51**

(51) Int Cl.:
**G06F 19/00** (2006.01)     **G01N 33/92** (2006.01)

(21) Application number: **08157988.0**

(22) Date of filing: **10.06.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicants:
- **Nederlandse Organisatie voor Toegepast-Natuurwetenschappelijk Onderzoek TNO 2628 VK Delft (NL)**
- **Universiteit Leiden 2311 EZ Leiden (NL)**

(72) Inventors:
- **Schalkwijk, Daniel Bernardus 3512 KB Utrecht (NL)**
- **Freidig, Andreas 3703 TC Zeist (NL)**

(74) Representative: **Hatzmann, Martin et al Vereenigde Johan de Wittlaan 7 2517 JR Den Haag (NL)**

(54) **Model system for diagnosing lipid metabolism**

(57)     The invention relates to a population model for the analysis of blood lipoprotein physiology in a test subject comprising:
a. a submodel for the production of blood lipoproteins;
b. a submodel for the lipolysis of blood lipoproteins;
c. a submodel for the reabsorption of blood lipoproteins; and

d. a submodel relating blood lipoprotein particle size to biochemical composition, more specifically triglyceride content,

thereby providing an analysis of the physiological processes underlying a steady state particle population distribution. Each submodel is given as function, using the size of the lipoprotein particle as the independent variable

Figure 1.

## Description

The invention relates to the field of mathematical models for lipid metabolism and use of these models in diagnosis of lipid related disease.

BACKGROUND

The importance of accurately measuring lipid levels in blood is well known. High levels of cholesterol in blood are known to increase the risk of a variety of diseases, such as atherosclerosis and related disorders such as cardiovascular events like stroke and high blood pressure, and metabolic syndrome, comprising a multitude of phenomena, like diabetes. In recent years it has appeared that the constitution of the blood lipids plays an important role in the development of and/or risk of lipid-related disorders.

Lipids are mainly present in the blood in the form of lipoproteins, which are spherical particles that transport cholesterol, triglycerides and other lipids in the bloodstream. Information on the sizes of the lipoprotein constituents is of major importance for a correct diagnosis of lipid-related diseases, but is normally not provided in traditional clinical diagnostics. Currently, several subclasses of lipoproteins are defined: VLDL (very low density lipoprotein) can be divided into 6 subclasses, LDL consists of 4 subclasses (including intermediate-density IDL) and HDL has been divided into 5 subclasses. It has appeared that pathological conditions or the risk theref relate to the amount and distribution of lipids over these subclasses. Based on these findings an 'atherogenic lipoprotein phenotype' has been defined, which takes into account a particle size profile within the LDL class (Austin, M.A. et al., 1990, Circulation 82:495-506). In addition to the cholesterol-based risk factors, apolipoprotein measurements such as the ApoB or the ApoB/ApoA-1 ratio have been found to indicate atherosclerosis risk (Alaupovic, P. 1996, Meth. Enzymol. 263:32-60; Walldius, G. et al., 2006, J. Intern. Med. 259:259-266).

Further improvements in risk assessment can only result from a more detailed understanding of lipoprotein physiology. To increase quantitative insight various multi-compartmental models have been developed to analyze experiments with radioactive or stable isotope labelled lipoprotein constituents. The first models describe the fluxes of apolipoprotein B between lipoprotein fractions (Fisher, W.R. et al., 1980, J. Lipid Res. 21:760-774), with subsequent refinements allowing better data interpretation (Fisher, W.R. et al., 1991, J. Lipid Res. 32:1823-1836; Packard, C.J. et al., 1995, J. Lipid Res. 36:172-187; Maistrom, R. et al., 1997, Arterioscler. Thromb. Vasc. Biol. 17:1454-1464). Other models describe the fluxes of triglycerides through the lipoprotein fractions (Harris, W.S. et al., 1990, J. Lipid Res. 31: 1549-1558; , Barrett, P.H. et al., 1991, J. Lipid Res. 32:743-762; Patterson, B.W. et al., 2002, J. Lipid Res. 43:223-233). Similar models describing other lipoprotein kinetics have also been developed (Campos, H. et al., 2001, J. Lipid Res. 42:1239-1249; Cohn, J.S. et al., 2004, J. Clin. Endocrinol. Metab. 89:3949-3955; Zheng, C. et al., J. Lipid Res. 48: 1190-1203). These models were developed to deal with various density-based lipoprotein separation techniques. Now, new measuring techniques such as HPLC and NMR measurements (e.g. US 5,343,389) provide more detailed size-concentration profiles of lipoproteins and their constituents.

The state-of-the art models that have been developed all are based on a so-called compartment model, or reactor model, which is a model in which compartments are defined with their own input and output. There is need for a model that more adequately describes and deals with the biological processes that form the basis of the lipoprotein metabolism.

SUMMARY OF THE INVENTION

The inventors now have developed a system that is able to reproduce a detailed size-concentration profile of blood lipoproteins and also size classes that correspond to experimental measurements. Examples include the classical VLDL1, VLDL2, IDL and LDL density classes. The model output includes steady state particle concentrations, but can also be applied to analyse fluxes of production, lipolysis and reabsorption at each particle size. The model is calculated deterministically, and specifies how the rate of each of the mentioned fluxes depends on the size of the lipoprotein.

More specifically, the system is presented as a population model for the analysis of blood lipoprotein physiology in a test subject comprising:

    a. a submodel for the production of blood lipoproteins;
    b. a submodel for the lipolysis of blood lipoproteins;
    c. a submodel for the reabsorption of blood lipoproteins; and
    d. a submodel relating blood lipoprotein particle size to biochemical composition, more specifically triglyceride content,

thereby providing an analysis of the physiological processes underlying a steady state particle population distribution.

In this model each submodel is given as function, using the size of the lipoprotein particle as the independent variable. Preferably, in the submodel for the lipolysis two models are contained, one for extra-hepatic tissue mediated lipolysis and one for hepatic lipolysis. Also preferably, the submodel for the reabsorption is able to distinguish between apoB and apoE mediated reabsorption.

Preferably, the population model according to the invention further comprises one or more of the following submodels:

  e. a submodel relating blood lipoprotein particle size to total cholesterol content;
  f. a submodel relating blood lipoprotein particle size to free cholesterol content;
  g. a submodel relating blood lipoprotein particle size to cholesterol ester content;
  h. a submodel relating blood lipoprotein particle size to phospholipid content;
  i. a submodel relating blood lipoprotein particle size to total protein content.

In more detail, the population model for the presence of blood lipoproteins in a test subject is a model wherein the total steady-state pool of lipoproteins $Q_{ss}$ in a diameter range $[d_a\ d_b]$ is given by:

$$Q_{ss}([d_a\quad d_b]) = \sum_{d_0}\sum_{d_i \in [d_a\quad d_b]} Q_{ss}^{d_0}(d_i)$$

wherein $Q_{ss}^{d_0}(d_i)$ is the steady state pool of a cascade step at diameter $d_i$ resulting from the particle influx at $d_0$. The steady-state pool in the cascade step $Q_{ss}^{d_0}(d_i)$ at each size $d$ resulting from the particle influx at $d_0$ is given by:

$$Q_{ss}^{d_0}(d_i) = J_{in}^{d_0}(d_i)\tau(d_i)$$

wherein $d$ is the particle diameter, $\tau$ signifies the residence time of a particle within a size class and $J_{in}^{d_0}(d_i)$ is the particle influx resulting from production if $d_i = d_0$ and from lipolysis otherwise.

Preferably in said model, the influx into the previous cascade step at particle diameter $d_{i-1}$ is defined as:

$$J_{in}(d_i) = J_{in}(d_{i-1})\frac{k_l(d_{i-1}) + k_{l,liver}(d_{i-1})}{k_l(d_{i-1}) + k_{l,liver}(d_{i-1}) + k_{u,liver}(d_{i-1})}$$

Where $k_{u,liver}$ is the particle reabsorption rate, $k_l$ is the extrahepatic lipolysis attachment rate, $k_{l,liver}$ is the hepatic lipolysis attachment rate and $d_{i-1}$ indicates the particle radius before the lipolysis step.

Also preferably, the residence time of a particle within a size class is given by

$$\tau(d_i) = \frac{1}{k_{a,liver}(d_i) + k_l(d_i)}$$

where $k_{a,liver}$ is the rate at which particles are attached to the liver to either be taken up or lipolysed *there*, $k_l$ is the lipolysis attachment rate for extrahepatic lipolysis.

Preferably, in the model of the invention the production of blood lipoproteins is given by the equation

$$J_{in}^{d_0}(d_0) = \begin{cases} J_{in,LDL} f_{nor}\left(d_0, \overline{d_{LDL}}, \sigma_{LDL}\right) & for & d_0 \in [d_{LDL\min}, d_{LDL\max}] \\ J_{in,IDL} f_{nor}\left(d_0, \overline{d_{IDL}}, \sigma_{IDL}\right) & for & d_0 \in [d_{IDL\min}, d_{IDL\max}] \\ J_{in,VLDL2} f_{nor}\left(d_0, \overline{d_{VLDL2}}, \sigma_{VLDL2}\right) & for & d_0 \in [d_{VLDL2\min}, d_{VLDL2\max}] \\ J_{in,VLDL1} f_{\log nor}\left(d_0, \mu_{VLDL1}, \sigma_{VLDL2}\right) & for & d_0 \in [d_{VLDL1\min}, d_{VLDL1\max}] \end{cases}$$

wherein do stands for the particle diameter at production, $\overline{d}$ stands for the average diameter in a given class, $\sigma$ is the standard deviation of the distribution curve. Subscripts indicate the class to which the diameter refers, and whether it is a minimum or maximum value for that class. $f_{nor}$ is the corrected normal probability density function, and $f_{lognor}$ is the corrected lognormal probability density function as follows:

$$f_{nor}(d, \overline{d}, \sigma) = \begin{cases} \dfrac{normpdf\ (d, \overline{d}, \sigma)}{normcdf\ (d_{\max}, \overline{d}, \sigma) - normcdf\ (d_{\min}, \overline{d}, \sigma)} & for & d > d_{\min} & and & d < d_{\max} \\ 0 & & & otherwise \end{cases}$$

and

$$f_{\log nor}(d, \mu, \sigma_{\ln}) = \begin{cases} \dfrac{\log normpdf(d - d_{\min}, \mu, \sigma_{\ln})}{\log normcdf(d_{\max} - d_{\min}, \mu, \sigma_{\ln})} & for & d > d_{\min} & and & d < d_{\max} \\ 0 & & & otherwise \end{cases}$$

wherein $normpdf(d, \overline{d}, \sigma)$ stands for a normal probability density function with parameters $\overline{d}$ and $\sigma$ evaluated at $d$, while $\log normpdf(d, u, \sigma_{\ln})$ stands for a lognormal probability density function with parameters $\mu$ and $\sigma_{\ln}$, evaluated at $d$.

Also preferably, in the model of the invention the lipolysis attachment rate in extra-hepatic tissue is given by the formula

$$k_{l,eht}(d) = \begin{cases} k_{l\max}\left(1 - \exp\dfrac{-(d - d_{l\min})^2}{2\sigma_l^2}\right) & for & d \geq d_{l\min} \\ 0 & & otherwise \end{cases}$$

wherein d is the particle diameter, $d_{lmin}$ is the minimum size at which lipolysis occurs, $k_{lmax}$ is the maximum lipolysis attachment rate and $\sigma_l$ is a shape parameter.

In a similar way, the lipolysis rate in the liver $k_{l,liver}$ can be given by the formula

$$k_{l,liver}(d) = k_{a,liver}(d) - k_{u,liver}(d)$$

wherein the liver attachment rate $k_{a,liver}$ is given either by

$$k_{a,liver}(d) = \begin{cases} k_{a,apoE\,max}\left(\dfrac{(d - d_{a,apoE\,min})\exp\dfrac{-(d - d_{a,apoE\,min})^2}{2(\sigma_{a,apoE} - d_{a,apoE\,min})^2}}{(\sigma_{a,apoE} - d_{a,apoE\,min})\exp\dfrac{-(\sigma_{a,apoE} - d_{a,apoE\,min})^2}{2(\sigma_{a,apoE} - d_{a,apoE\,min})^2}}\right) + k_{a,apoB} & for \quad d \geq d_{a,apoE\,min} \\ k_{a,apoB} & otherwise \end{cases}$$

or by

$$k_{a,liver}(d) = \begin{cases} k_{a,apoE\,max}\left(\dfrac{f_{weibullpdf}\left(d - d_{a,apoE\,min}, A_{a,apoE\,min}, B_{a,apoE\,min}\right)}{f_{weibullpdf\,max}}\right) + k_{a,apoB} & for \quad d \geq d_{a,apoE\,min} \\ k_{a,apoB} & otherwise \end{cases}$$

and the liver uptake rate $k_{u,liver}$ is given by

$$k_{u,liver}(d) = \begin{cases} (k_{a,liver} - k_{a,apoB})\left(1 - \exp\dfrac{-(d - d_{a,apoE\,min})^2}{2\sigma_{u,liver}^2}\right) + k_{a,apoB} & for \quad d \geq d_{a,apoE\,min} \; \mathbf{w} \\ k_{a,apoB} & otherwise \end{cases}$$

herein $k_{a,apoEmax}$ is the maximum liver uptake rate due to apoE-mediated reabsorption, $k_{a,apoB}$ is the liver uptake rate due to apoB-mediated reabsorption, d is the particle diameter, $d_{a,apoEmin}$ is the minimum particle diameter at which liver lipolysis takes place and $\sigma_{a,apoE}$ and $\sigma_{u,liver}$ are shape parameters and where $f_{weibullpdf}(d,A,B)$ is a weibull probability density function evaluated at d with shape parameters A and B, and $f_{weibullpdfmax}$ is the maximum function value attained by this weibull function on the lipoprotein size range from 0 attained by this weibull function on the lipoprotein size range from 0 to 200 nm.

In another embodiment, the invention provides a method to determine individual parameters of each submodel using data obtained from a blood sample in a subject comprising:

 j. taking a blood sample from said subject;
 k. providing a data set from said sample comprising either the number of blood lipoprotein particles in a size class or the chemical composition of said particles, wherein at least 6 size classes are provided;
 l. feeding said data to a model according to the invention;
 m. finding parameters for the submodels defined in the model of the invention such that the resulting calculated total steady-state pool of lipoproteins $Q_{ss}$ for every diameter $d$ is in agreement with said dataset.

Preferably in said method additionally from said sample or said subject one or more data are provided, including but not limited to data from the group consisting of the ApoC3 content, the ApoA5 content, insulin sensitivity indexes, the sialic acid content, the lipoprotein lipase activity, the hepatic lipase activity, the content of C-reactive protein, the content of adiponectin, gene expression data in blood cells, relevant single nucleotide polymorphisms and copy number variations.

In a further embodiment the invention comprises a method to monitor the development of disease or the effect of a therapy in a patient by performing the method of the invention.

Preferably in the above methods said disease is selected from the group of lipid metabolism disorders, including but not limited to hyper-and hypocholesterolemia, hypertriglyceridemia and hyperlipoproteinemia types I, IIa, IIb, III, IV and V.

In a further embodiment, the invention comprises the use of the method according to the invention to choose a patient specific therapeutic intervention, directed at one or more processes that are described by one or more sub models relating to composition, production lipolysis and reabsorption of lipoproteins.

LEGENDS TO THE FIGURES

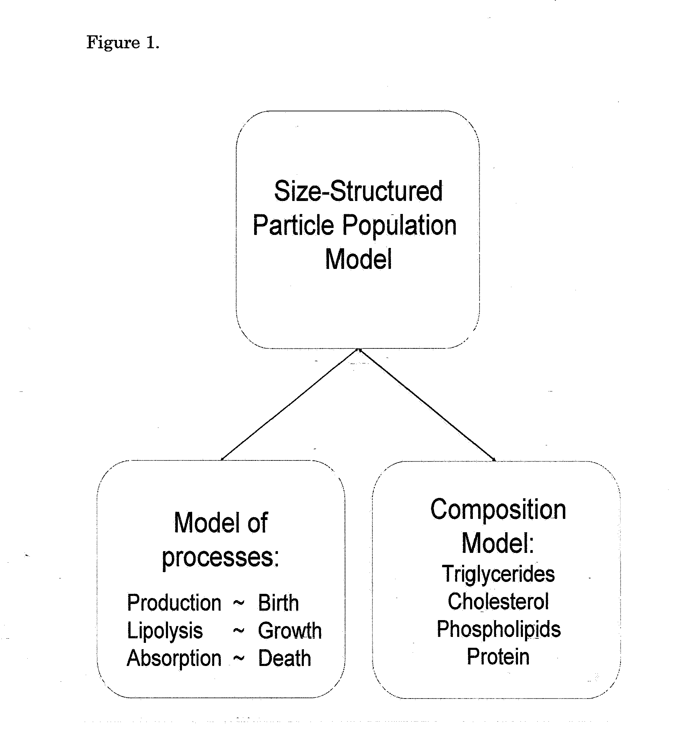

Figure 1: The model framework consists of three modules. In the Kernel module, a size-structured simulator is used to model the particle population and assess the steady-state model prediction. The first sub-model describes how lipoprotein affinity for the modelled physiological processes depends on particle size. The second sub-model describes how the lipoprotein's biochemical composition depends on particle size. Each module can be modified separately.

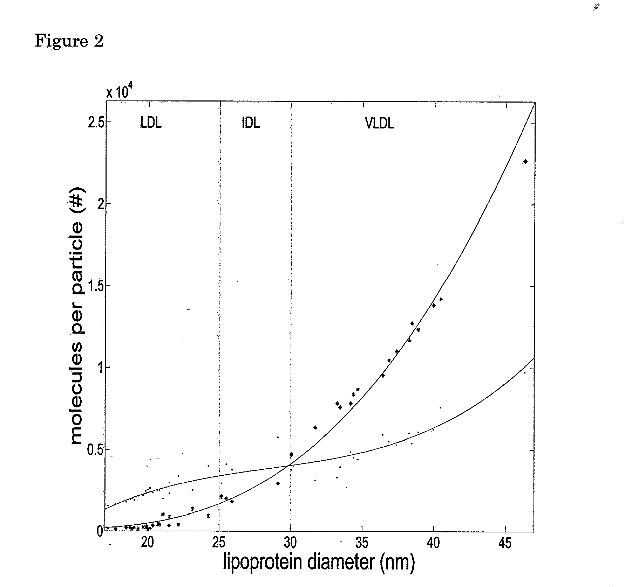

Figure 2: Representation of the empirical size-composition submodel, based on data from Tuzikov et al. (2002, Voprosy Meditsinskoj Khimii, 48:90-91). From these data the number of triglyceride (*) and cholesterol (·) molecules per lipoprotein particle was calculated. The triglyceride data was fitted with a sixth order polynomial, the cholesterol data with a third order polynomial.

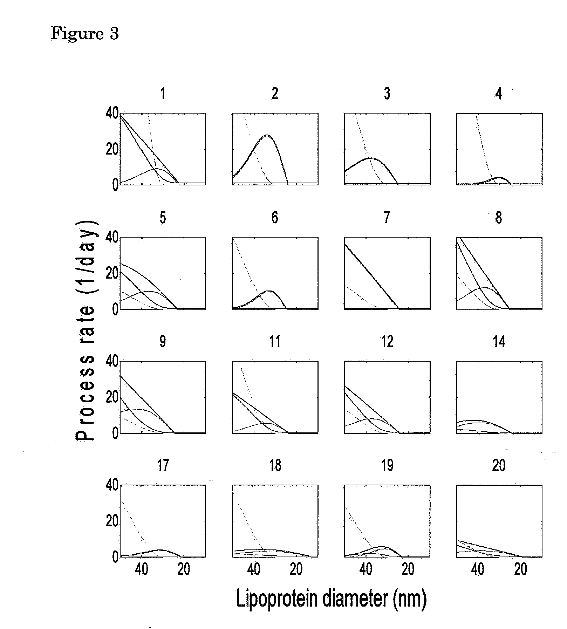

Figure 3: How the rate of each process depends on particle size, based on the fitted parameters shown in Table 1. The patient number is indicated above each subfigure. The processes shown are extrahepatic (LpL-mediated) lipolysis (green), liver attachment (black), liver (HL-mediated) lipolysis (red) and liver uptake (blue). Clear differences between patients are observed.

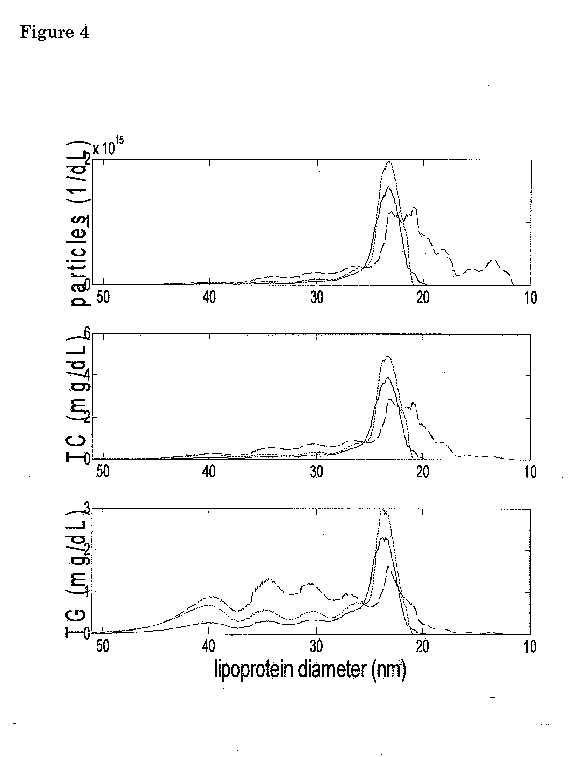

Figure 4: Average particle, total cholesterol and triglyceride concentrations of model fits based on flux data of VLDL1, VLDL2, IDL and LDL only. The three lines represent averages of the subjects in the three phenotype groups as determined by Packard et al. (2000, J. Lipid Res. 41:305-318). The solid line indicates phenotype 'A' (LDL peak size > 26 nm), the dotted line phenotype 'I' (LDL peak size between 25 and 26 nm) and the striped line phenotype 'B' (LDL peak size< 25 nm). Although the data did not contain any particle size information further than the four mentioned classes, the model does reproduce the peak size shift in the LDL size range.

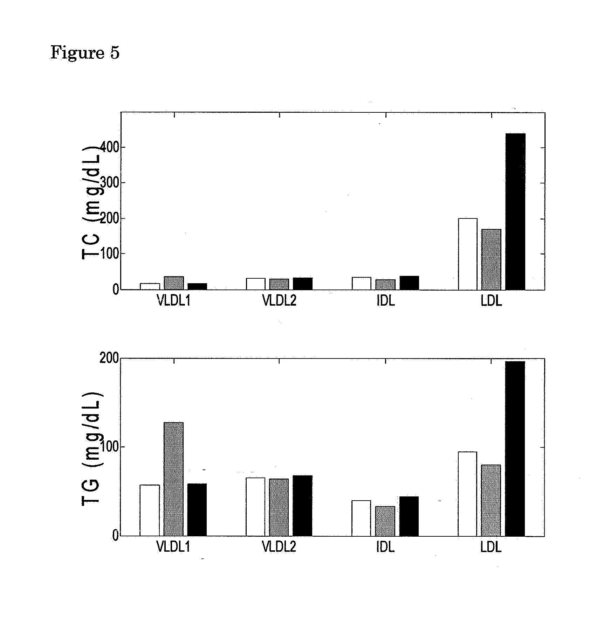

Figure 5: Total Cholesterol and Triglyceride concentration in model fit for patient 19 (white bars), a simulated lipolysis polymorphism reducing the lipolysis affinity (grey bars) and a simulated apoB-related reabsorption polymorphism, reducing the ApoB-related reabsorption affinity (black bar). The lipolysis polymorphism specifically increases VLDL1 triglycerides. The apoB-related reabsorption polymorphism increases LDL cholesterol. Both simulations are in accordance with observed phenotypes.

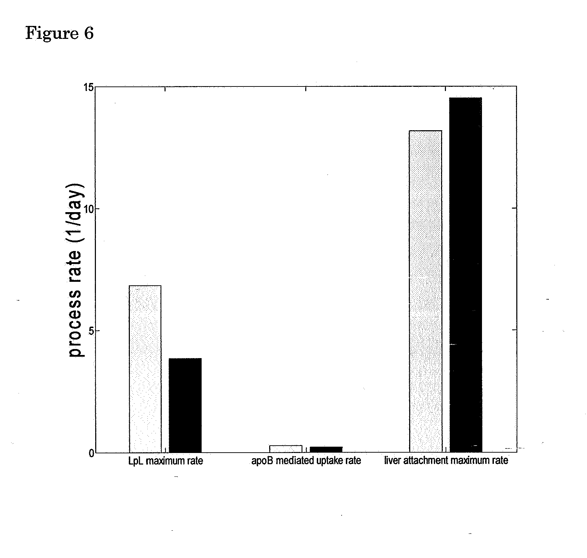

Figure 6: Rate parameter values of two patients estimated based on 6 subclasses derived from measurements by Liposearch. The patients clearly differ in their individual parameters.

DETAILED DESCRIPTION OF THE INVENTION

The inventors now are proposing a population model for the distribution of blood lipoproteins in which biological processes like production of lipoproteins, reabsorption of lipoproteins and lipolysis influence the size-dependent distribution of lipoproteins. In this way the steady-state values of the lipoprotein content of a certain size class can be predicted. For this, the model consists of a kernel and submodels, shown schematically in figure 1. The kernel consists of a size-structured lipoprotein population model which calculates the steady-state lipoprotein levels at different particle sizes. The first submodel (in fact consisting of three submodels) specifies the particle's lifecycle processes of production, lipolysis and reabsorption in a particle-size dependent fashion. The second submodel specifies the relation between particle size and biochemical composition, which is estimated based on empirical data.

**Population Model**

The kernel of the model framework, the size-structured lipoprotein population model, calculates the steady-state lipoprotein concentration along the size spectrum of ApoB-containing lipoproteins, which ranges from approximately 10 to 100 nm. The steady-state concentration is calculated using information on the production, lipolysis and reabsorption processes affecting single lipoprotein particles from the first submodel.

Model calculation proceeds as follows. The model uses the production into each class's small size interval as input and calculates the ensuing 'cascades' of lipolysis steps. These steps make the particles successively smaller until the minimum size at which lipolysis can occur is passed. The size of the cascade steps is determined by a model parameter specifying the triglyceride fraction a particle loses during lipolysis. The relation between triglyceride content and particle size is made by the second submodel, described below. During the lipolysis cascade, part of the lipoproteins is lost due to reabsorption processes. Once all the processes are specified, the resulting concentration at each cascade step is calculated and associated to the corresponding average particle size. The final particle concentration in the system is found by summing the concentrations of all the overlapping instances of different cascades that fall within a certain class's size region.

After a particle is produced at a given size, its subsequent sizes are determined by the size step due to lipolysis. Using the assumption that the fraction of triglycerides lost $f_{tg}$ is constant at each step, this is given by:

$$n_{tg}\left(d_{i+1}\right) = (1 - f_{tg})n_{tg}\left(d_i\right)$$

where $n_{tg}(d_i)$ is the initial and $n_{tg}(d_{i+1})$ the final number of triglyceride molecules in a lipoprotein particle, as a function of particle diameter. The corresponding particle diameter is given by the second submodel below.

The equation for the total steady-state pool $Q_{ss}$ in a given diameter range $[d_a\ d_b]$ is given by:

$$Q_{ss}\left(\left[d_a \quad d_b\right]\right) = \sum_{d_0} \sum_{d_i \in [d_a \quad d_b]} Q_{ss}^{d_0}(d_i)$$

where $Q_{ss}^{d_0}(d_i)$ is the steady state pool of a cascade step at diameter $d_i$ resulting from the particle influx at $d_0$. The

steady-state pool in the cascade step $Q_{ss}^{d_0}(d_i)$ at each size $d$ resulting from the particle influx at $d_0$ is given by:

$$Q_{ss}^{d_0}(d_i) = J_{in}^{d_0}(d_i)\tau(d_i)$$

where $d$ is the particle diameter, $\tau$ signifies the residence time of a particle within a size class and $J_{in}^{d_0}(d_i)$ is the particle

influx resulting from production if $d_i = d_0$ and from lipolysis otherwise. The influx from lipolysis in steady state depends on the influx into the previous cascade step at particle diameter $d_{i-1}$, as follows:

$$J_{in}(d_i) = J_{in}(d_{i-1})\frac{k_l(d_{i-1}) + k_{l,liver}(d_{i-1})}{k_l(d_{i-1}) + k_{l,liver}(d_{i-1}) + k_{u,liver}(d_{i-1})}$$

Here $k_{u,liver}$ is the particle reabsorption rate, $k_l$ is the extrahepatic lipolysis attachment rate, $k_{l,liver}$ is the hepatic lipolysis attachment rate and $d_{i-1}$ indicates the particle radius before the lipolysis step. The residence time of a particle within a size class is given by:

$$\tau(d_i) = \frac{1}{k_{a,liver}(d_i) + k_l(d_i)}$$

Here $k_{a,liver}$ is the rate at which particles are attached to the liver to either be taken up or lipolysed there, $k_l$ is the lipolysis attachment rate for extrahepatic lipolysis. The formulae for these diameter-dependent processes are specified by the first submodel, described below.

Model calculation proceeds using a discretisation of the size range into different classes. The mean size of the range of the class is used as a starting point for a cascade, and all subsequent cascade steps are added to the corresponding class. The number of classes can be chosen at will.

The model output can be given in various forms. The model can reproduce a detailed size - concentration profile, but also size classes that correspond to experimental measurements. Examples include the classical VLDL1, VLDL2, IDL and LDL density classes, and concentration - size profiles measured by HPLC or NMR (e.g. US 5,343,389). The model output includes steady state particle concentrations, as well as fluxes of production, lipolysis and reabsorption at each particle size.

**Process submodel**

The size-dependent models for production, reabsorption and lipolysis are based on biological hypotheses. The hypotheses were translated into mathematical equations. The current model can be can be considered a first functional approximation, to which further biological knowledge can be added in order to arrive at more detailed analysis of lipoprotein physiology.

**Production** The speed of the production process in each class is based directly on the studied dataset. The size distribution within a size class is based on biological considerations. Production of ApoB-100 VLDL particles is thought to be a two-step process, in which first VLDL2 is produced intercellularly, which can subsequently be fused to a lipid droplet to form VLDL1. This idea was translated to the model by assuming a normal size distribution of secreted particles within the VLDL2, IDL and LDL ranges, since these are expected to vary around a given mean. For the VLDL1 range a lognormal size distribution was assumed, since the size of the fused lipid droplets can vary greatly. The mean of the VLDL 2 fraction was derived from the TG to ApoB ratio of the production in these classes presented by Adiels et al. (2005, J. Lipid Res. 46:58-67). The mean of the VLDL 1 fraction was fitted as a model parameter. Since for IDL and LDL no data were available, the class middle was taken as distribution mean. The standard deviation of the curves was taken as half the distance from the distribution mean to the nearest class border. For the VLDL 1 class this rule applied to the expectation and the square root of the variance of the lognormal production distribution, as specified in the Examples. A correction was applied to ensure all produced particles in each class actually fell inside the specified class.

Mathematically this can be expressed as:

$$J_{in}^{d_0}(d_0) = \begin{cases} J_{in,LDL} f_{nor}\left(d_0, \overline{d_{LDL}}, \sigma_{LDL}\right) & for \quad d_0 \in [d_{LDL\,min}, d_{LDL\,max}] \\ J_{in,IDL} f_{nor}\left(d_0, \overline{d_{IDL}}, \sigma_{IDL}\right) & for \quad d_0 \in [d_{IDL\,min}, d_{IDL\,max}] \\ J_{in,VLDL2} f_{nor}\left(d_0, \overline{d_{VLDL2}}, \sigma_{VLDL2}\right) & for \quad d_0 \in [d_{VLDL2\,min}, d_{VLDL2\,max}] \\ J_{in,VLDL1} f_{\log nor}\left(d_0, \mu_{VLDL1}, \sigma_{VLDL2}\right) & for \quad d_0 \in [d_{VLDL1\,min}, d_{VLDL1\,max}] \end{cases}$$

where $d_0$ stands for the particle diameter at production, $\overline{d}$ stands for the average diameter in a given class, σ is the standard deviation of the distribution curve. Subscripts indicate the class to which the diameter refers, and whether it is a minimum or maximum value for that class. The different $J_{in}$'s are production rates in each class, which are estimated based on data (Packard et al. 2000, J. Lipid Res. 41:305-318). $f_{nor}$ is the corrected normal probability density function, and $f_{lognor}$ is the corrected lognormal probability density function starting at $d = d_{VLDL1min}$. These are cut off at the class borders, and corrected for the fraction falling outside, so that all production falls into the specified class. This was specified as follows for the corrected normal probability density function:

$$f_{nor}(d, \overline{d}, \sigma) = \begin{cases} \dfrac{normpdf\left(d, \overline{d}, \sigma\right)}{normcdf\left(d_{max}, \overline{d}, \sigma\right) - normcdf\left(d_{min}, \overline{d}, \sigma\right)} \\ 0 \end{cases}$$

*for $d > d_{min}$ and $d < d_{max}$ otherwise* and as follows for the corrected lognormal probability density function:

$$f_{\log nor}(d, \mu, \sigma_{\ln}) = \begin{cases} \dfrac{\log normpdf\left(d - d_{min}, \mu, \sigma_{\ln}\right)}{\log normcdf\left(d_{max} - d_{min}, \mu, \sigma_{\ln}\right)} \\ 0 \end{cases}$$

*for $d > d_{min}$ and $d < d_{max}$ otherwise*

wherein $normpdf(d, \overline{d}, \sigma)$ stands for a normal probability density function and $normcdf(d, \overline{d}, \sigma)$ for a normal cumulative density function with parameters $\overline{d}$ and σ evaluated at d. While $lognormpdf(d, \mu, \sigma_{\ln})$ stands for a lognormal probability density function and $\log normcdf(d, \mu, \sigma_{\ln})$ or a lognormal cumulative density function with parameters μ and $\sigma_{\ln}$, evaluated at $d$.

Possible values for $\overline{d}$ and σ can be found in the following table

| Class | $\overline{d}$(nm) | σ(nm) |
|---|---|---|
| LDL | $\dfrac{25-d_{LDL\min}}{2}$ | $\dfrac{\overline{d}_{LDL}-d_{LDL\min}}{2}$ |
| IDL | 27.5 | 1.25 |
| VLDL2 | 33.54 | 1.77 |
| VLDL1 | 41.87 | 2.94 |

wherein $d_{LDLmin}$ is derived from the free model parameters. It is one lipolysis step smaller than the smallest possible lipolysis attachment size. The figures in the table represent the values that give the best result in the current model, based on extensive testing as is shown in the Examples.

The formula for translating the expectation $\overline{d_{VLDL1}}$ and standard deviation σ for the VLDL 1 class particle diameter to the mean (μ) and standard deviation ($σ_{ln}$) of a lognormal distribution is given by:

$$\mu_{VLDL1} = \ln\left(\overline{d_{VLDL1}} - d_{VLDL1\min}\right) - \frac{1}{2}\ln\left(1 + \frac{\sigma^{2}_{VLDL1}}{(\overline{d_{VLDL1}} - d_{VLDL1\min})^{2}}\right)$$

$$\sigma_{ln}^{\;2} = \ln\left(1 + \frac{\sigma^{2}_{VLDL1}}{(\overline{d_{VLDL1}} - d_{VLDL1\min})^{2}}\right)$$

**Extrahepatic tissue - lipolysis** In the extrahepatic tissue particles are only lipolysed, reabsorption of particles is negligible (Lichtenstein, L. et al., 2007, Arterioscler. Thromb. Vasc. Biol. 27:2420-2427). Lipolysis of lipoproteins in extrahepatic tissue is carried out mainly by lipoprotein lipase (LpL). This enzyme mainly lipolyses larger lipoproteins such as VLDL 1, while VLDL 2 and IDL are lipolyzed to a subsequently lesser extent (Demant, T. et al., 1993, J. Lipid Res. 34:147-156). The particle binds to cell-surface heparin sulphate proteoglycans (HSPG's) mainly through LpL itself, while apoE modulates the binding affinity (de Beer, F. et al., 1999, Arterioscler. Thromb. Vasc. Biol. 19:633-637). Multiple LpL's which were already bound to the HSPG's can then be transferred to the lipoprotein, and mediate the lipolysis of the particle. What exactly determines the speed of this lipolysis is not known, although the available surface area, apolipoprotein C regulation and the biochemical composition of the particle are all hypothesized to influence the speed (Adiels, M, 2004, PhD thesis, Chalmers Univ. Technol., Göteborg, Sweden). In the model framework the lipolysis process is split into two steps: the first step decides whether a particle is bound to a HSPG for lipolysis, the second how many triglycerides it loses during lipolysis. The first step depends on the particle's attachment affinity to HSPG's, which in turn depends on its apolipoprotein composition. This means that the total affinity of the particle for HSPG increases with particle size, until a maximum is reached. Since the exact change in affinity with particle size is not known, this is approximated by a flexible one-parameter function with a shape similar to what would be expected. For this purpose a cumulative density function of the Rayleigh distribution was chosen. The formula for the lipolysis attachment rate in extrahepatic tissue $k_{l,eht}(d)$ then becomes,

$$k_{l,eht}(d) = \begin{cases} k_{l\max}\left(1 - \exp\dfrac{-(d - d_{l\min})^2}{2\sigma_l^2}\right) \\ 0 \end{cases}$$

*for $d \geq d_{l\min}$ otherwise*

where $d$ is the particle diameter, $d_{l\min}$ is the minimum size at which lipolysis occurs, $k_{l\max}$ is the maximum lipolysis attachment rate and $\sigma_l$ is a derived shape parameter.

**Liver - lipolysis and uptake** In the liver the particle first needs to be attached to liver HSPG's. This process is primarily mediated by apoE, so that the attachment does not work for LDL particles without apoE. These LDL particles only contain apoB, which can attach to the LDL receptor and lead to the reabsorption of small particles. Larger particles can either be lipolysed or be reabsorbed. The lipolysis in the liver is primarily mediated by hepatic lipase (HL), an enzyme which functions primarily on smaller apoB and apoE-containing lipoproteins such as IDL, and to a lesser extent on VLDL 2 (Demant, T. et al. 1988, J. Lipid Res. 29:1603-1611). Reabsorption of larger lipoproteins can take place via LRP. Roles for SRB1 and direct incorporation via HSPGs have also been suggested (Out, R. et al., 2004, J. Biol. Chem. 279: 18401-18406; MacArthur, J. et al., 2007, J. Clin. Invest. 117:153-164).

In the model liver attachment and further processing are again described as a two-step process. First attachment takes place, mainly mediated by apoE, but with a small contribution from apoB. Although small, this contribution is important especially in the LDL size range, where a small uptake affinity combined with large amounts of particles result in a considerable uptake flux. Since attachment increases and can subsequently decrease with particle size, a Rayleigh probability density function is used to describe this pattern. In order to have its maximum at one, it is scaled using the maximum of this same function, which lies at $d = \sigma_{a,apoE}$. The liver attachment rate $k_{a,liver}$ can either be given by

$$k_{a,liver}(d) = \begin{cases} k_{a,apoE\max}\left(\dfrac{(d - d_{a,apoE\min})\exp\dfrac{-(d - d_{a,apoE\min})^2}{2(\sigma_{a,apoE} - d_{a,apoE\min})^2}}{(\sigma_{a,apoE} - d_{a,apoE\min})\exp\dfrac{-(\sigma_{a,apoE} - d_{a,apoE\min})^2}{2(\sigma_{a,apoE} - d_{a,apoE\min})^2}}\right) + k_{a,apoB} \\ k_{a,apoB} \end{cases}$$

*for $d \geq d_{a,apoE\min}$ otherwise* or instead by

$$k_{a,liver}(d) = \begin{cases} k_{a,apoE\max}\left(\dfrac{f_{weibullpdf}(d - d_{a,apoE\min}, A_{a,apoE\min}, B_{a,apoE\min})}{f_{weibullpdf\max}}\right) + k_{a,apoB} \\ k_{a,apoB} \end{cases}$$

*for $d \geq d_{a,apoE\min}$ otherwise*

Subsequently, part of the attached lipoproteins are taken up. Since apoB attachment mainly occurs through the LDL receptor all apoB-mediated attachment results in uptake, the part attached through apoE can result in lipolysis. Since HL mainly lipolyses smaller particles the fraction of lipolyzed lipoproteins increases with decreasing particle size. The resulting liver uptake rate $k_{u,liver}$ then becomes.

$$k_{u,liver}(d) = \begin{cases} \left(k_{a,liver} - k_{a,apoB}\right)\left(1 - \exp\dfrac{-(d - d_{a,apoE\,\min})^2}{2\sigma^2_{u,liver}}\right) + k_{a,apoB} \\ k_{a,apoB} \end{cases}$$

*for $d \geq d_{a,apoE\,\min}$* otherwise

The lipolysis rate in the liver $k_{l,liver}$ is then given by:

$$k_{l,liver}(d) = k_{a,liver}(d) - k_{u,liver}(d)$$

In these equations $k_{a,apoEmax}$ is the maximum liver uptake rate due to apoE-mediated reabsorption, $k_{a,apoB}$ is the liver uptake rate due to apoB-mediated reabsorption, $d$ is the particle diameter, $d_{a,apoEmin}$ is the minimum particle diameter at which liver lipolysis takes place and $\sigma_{a,apoE}$ and $\sigma_{u,liver}$ are shape parameters. Furthermore, $f_{weibullpdf}(d,A, B)$ is a weibull probability density function evaluated at $d$ with shape parameters A and B, and $f_{weibullpdfmax}$ is the maximum function value attained by this weibull function on the lipoprotein size range. Note that in this case the probability functions are not used as such, but rather to indicate the shape of the rate distribution over lipoprotein particle sizes.

**Size - composition submodel**

The relation between particle diameter and both particle cholesterol and triglyceride content was based on a dataset presented by Tuzikov et al. (2002, Voprosy Meditsinskoj Khimii 48:90-91). The size - triglyceride relationship was fitted using a sixth degree polynomial, the size-cholesterol relationship by a third-degree polynomial, shown in figure 2. Although it is known that the mapping between particle size and biochemical composition is not always one-one, the current approach gives a solid first approximation which may still be improved in future versions of the model. A second option is to use the same model that Tuzikov *et al*. presented.

It is further envisaged, that similar submodels for the relation between particle size and total or free cholesterol content, for particle size and cholesterol ester content, for particle size and phospholipid content and for particle size and total protein content can be developed.

As is shown in the Examples the presently proposed model is capable of predicting the size distribution (population) of blood lipoproteins. The model can also analyse a measured size distribution, and derive parameters that indicate the status of the production, lipolysis and reabsorption processes. In practice the model can calculate this output with minimum input data, as compared to the state-of-the-art models.

Clinical data sets are obtained from a blood sample of an individual patient. Each data set should consist of, at least, a size distribution profile of ApoB containing lipoproteins in plasma where the distribution is separated in 6 or more classes and where the size range of each class can be expressed in nm. The distribution profile needs to provide information on either the number of lipoprotein particles per class or on the chemical composition of particles found within the class (such as triglyceride and cholesterol concentration in a class). Advantageously, additional clinical chemical data from the blood sample or from subfractions of the sample can be used to improve the diagnostic parameters of the model, such as the content of ApoC3 and Apo A5, the insulin sensitivity (e.g. HOMA index), the sialic acid content, lipoprotein lipase activity, hepatic lipase activity, C-reactive protein (CRP), adiponectin levels, gene expression data in blood cells and data on genetic background including relevant single-nucleotide polymorphisms (SNP's) and copy number variations.

The model is particularly useful to monitor progress of a disease or a therapy for a disease, where the disease is known to influence the blood lipoprotein distribution and/or content. Such diseases are for instance hypertriglyceridemia, hypercholesteremia and hyperlipoproteinemia types I, IIa, IIb, III, IV and V.

The model is available in an executable computer program, which can be made functional on any personal computer. As such, a digital carrier with the program instructions forms part of the present invention. This digital carrier can be a diskette, a hard disk, a memory stick and the like.

The program needs as input clinical data form a blood sample of an individual patient, as had been specified above, essentially comprising a size distribution of ApoB containing lipoproteins where the distribution is separated in 6 or more classes. Optionally additional clinical data can be entered. Furthermore, of course, specifics about the sample itself and the patient from who it is derived are registered.

The output of the program is a graph representing the steady-state distribution of blood lipoproteins and optionally the output can comprise a description of any aberrant physical processes that underlie the resulting information, a proposal for a diagnosis and/or a therapy or a comparison on the severity or progress with any previously obtained results from the same patient. Thus, the model can be of great assistance for the clinician in diagnosis and therapy of patients having a disease relating to changes in the blood lipoprotein profile.

Further, the model can be of use in the study of diseases related to blood lipoproteins and factors affecting the metabolism of blood lipoproteins.

EXAMPLES

In order to test the model's capability to reproduce measured lipoprotein flux data, the model was fitted to data from a stable isotope labeling study by Packard et al. (2000, J. Lipid Res. 41:305-318).
The individual patient's model outcomes of this study were used as input to the current model. Packard and coworkers divided their subjects into three groups based on the 'LDL peak size'. Phenotype 'A' had an LDL peak size greater than 26 nm, phenotype 'I' an LDL peak size between 25 and 26 nm and phenotype 'B' an LDL peak size smaller than 25 nm.
In Packard et al. (2000, J. Lipid Res. 41:305-3-18) the flux data are analyzed by a multi-compartment model to reveal the pool size of each lipoprotein density fraction, as well as the influx from the previous class into the reported class, here interpreted as the lipolysis flux, and the direct catabolism from the fraction, here presented as reabsorption. Also the direct production into each class is quantified. The dataset was considered to be in steady state when the total influx into each category given by production plus lipolysis influx equals the total efflux given by reabsorption plus lipolysis efflux. Datasets in which a large imbalance between total input and total output in one class were found were disregarded, leading to the exclusion of four patients. This selection is necessary because the current model assumes steady-state, which therefore needs to be present in the data. Since the original paper separated the VLDL1, VLDL2, IDL and LDL categories, the model was adapted to reproduce these size classes.
The deviation between the modelled and measured pool sizes is calculated as an average percent difference per datapoint. For the fluxes, this measure is not possible since the data contain several zero entries. Therefore an alternate measure was devised which sums the deviations of all modelled and measured data points, and divides them by the summed flux of the process, also giving a percentage score. Since the model parameters are most sensitive to an accurate pool size fit, the pool size fit was given double the importance of the average fluxes fit. In formula:

$$D = \frac{4}{6} \cdot \sum_i \frac{n_i^d - n_i^m}{n_i^d} + \frac{1}{6} \frac{\sum_i J_i^{l,d} - J_i^{l,m}}{\sum_i J_i^{l,d}} + \frac{1}{6} \frac{\sum_i J_i^{r,d} - J_i^{r,m}}{\sum_i J_i^{r,d}}$$

Where $D$ stands for deviation, $n$ indicates the pool size, superscript $d$ indicating the data and $m$ the model fit. $J$ stands for a flux, superscripts $d$ and m as before, $l$ indicates lipolysis, $r$ indicates reabsorption. Subscript $i$ indexes the different data points of each lipolysis size class.
The dataset of Packard et al contains estimations for the apoB in lipoprotein pools of the various classes in mg, and turnover speeds in pools per day. These are converted to particle concentrations and particle fluxes respectively. This needs the assumption that only ApoB-100 is present on lipoprotein particles in the fasted state.

$$n\left(\frac{mol}{L}\right) = \frac{n(g)}{M_{ApoB-100}(g/mol) \cdot V_{blood}(L)}$$

Where n is the number of lipoproteins, $M_{ApoB-100}$ the molar mass of ApoB-100 and $V_{blood}$ the blood volume of a person (taken to be 5 L).
The resulting fitted profile can be viewed in as much detail as is required. This allows the comparison of the modelled 'LDL peak size' with the patient's LDL peak size class based on measurements.
The data from the study by Packard et al. (2000, J. Lipid Res. 41:305-318) were fitted using the Nelder-Mead Simplex Method (implemented in Matlab as the function fminsearch) for optimizing the parameters of the model. The deviation score used is given above. The Simplex was run from sixty-four starting points which were specified by taking two extreme values for each parameter and using a full 'experimental design'. Subsequently the three best fitting parameter sets were selected and the Simplex was applied iteratively to them, until the parameter set did not change. These three final parameter sets were then compared. If the final parameters were found to differ, the whole procedure was repeated using the minima and maxima of each parameter in the set of final parameters as starting points for a new experimental design.

It is also possible to inspect the differences in fitted parameters between the groups defined by Packard *et al*. using the nonparametric Kruskal-Wallis test. This test is suitable for small datasets and does not need the normality assumption a one-way Anova would require. It compares the medians of the parameters describing the three groups of patients.

The fitted model parameters give some information about the processes making up the final lipoprotein profile. To aid interpretation, various process-indicating parameters can be derived from the fitted model parameters. These can either be process indicators, such as the maximum HL activity, the particle size at which it HL affinity is at a maximum and the average apoE-related uptake affinity over the VLDL1 range. They can also be size-class specific indicator parameters of process, age or size averages per particle in that class. For example, the average lipolysis attachment rate per particle in the VLDL1 size class may be calculated. This differs from the 'transfer from VLDL1 to VLDL2' variable presented by Packard and coworkers, since it takes into account all lipolysis steps of VLDL1 particles, also those that do not cause the particle to change class.

The model's potential for modeling biological polymorphisms was investigated. Two defects were simulated. The first is a polymorphism in the ApoB-related reabsorption which leads to hypercholesterolemia, the second a defect in LpL lipolysis, which leads to hypertriglyceridemia. Data from Patient "19" in Packard et al (2000, J. Lipid Res. 41: 305-318) was chosen for the in-silico experiment. This patient is in the 'B' category, with low LDL peak size, and higher CVD risk. The profile of the patient was compared with the same profile firstly if the ApoB-related reabsorption activity was halved, corresponding to an LDL-receptor polymorphism. This was simulated by setting the $k_{a,apoB}$ parameter to half its original value. Secondly the LpL-mediated lipolysis activity was reduced, corresponding to an LpL defect. This was simulated by setting the $k_{lmax}$ value to 25% of its original value. The output of the model is reproduced for the LDL, IDL, VLDL1 and VLDL2 size classes.

Finally, the model was applied to lipoprotein subclass data from a single blood sample. The data were measured by the company Liposearch. The reported cholesterol and triglyceride data at various particle sizes were converted to particle concentrations as follows.

$$V_{core} = \frac{4\pi}{3} \left( \overline{d} - d_{shell} \right)^3$$

$$M_{tg,core} = \frac{\dfrac{C_{tg}}{\rho_{tg}}}{\dfrac{C_{tg}}{\rho_{tg}} + \dfrac{f_{ce}C_{tc}}{\rho_{ce}}} \rho_{tg} V_{core}$$

$$n_{part} = \frac{C_{tg}}{M_{tg,core}}$$

wherein $V_{core}$ is the core volume of the lipoprotein particle at average particle size in a class $d$. $M_{tg,core}$ is the triglyceride content of the core in a given class, $C_{tg}$ is the measured triglyceride concentration, $\rho_{tg}$ is the triglyceride density taken to be 0.92 g / cm³, $\rho_{ce}$ is the cholesterol ester density taken to be 0.95 g /cm³ and $f_{ce}$ is the fraction of cholesterol ester versus free cholesterol, based on the biochemical submodel presented above. Finally $n_{part}$ is the number of particles in a specified class.

The dimensionality of the data was reduced to six datapoints, by taking together the large VLDL subfractions, the medium and small VLDL subfractions and the very small LDL subfractions and leaving the large medium and small LDL subfractions as reported. The model, using the second option for the liver attachment function, was fitted to this data.

*Results*

**Feasibility of Model Approach**

The pool and flux data were well fitted with the model. In eleven patients the model fit converged to a difference of less then 1% between parameters in the three best fit parameter sets. A difference of less then 10% was found in all but one patient. Only patient 17 clearly converged to two local minima, which differed only slightly in the deviation score. The minimum with the smallest deviation score was chosen. Table 1 shows the parameters that have been estimated for all subjects from the study by Packard et al. and the corresponding deviations, the definition of which can be found in above. Figure 3 shows how the processes vary with particle size, given these parameters. The deviation ranged from 1.5% to 19.4% with an average of 7%. Patients 4, 8 and 18 have the highest deviations, without them the average deviation is 4.9%. It is striking that these patients have high uptake in both the LDL and VLDL1 classes, but very low uptake in the intermediate IDL and VLDL2 classes. The current model was not able to reproduce this curious pattern. Further investigation into the underlying kinetic data could reveal whether this is a physiological phenomenon, or an artifact of Packard's first model analysis of the data. The model analysis shows that the current model could reproduce flux data.

| Subj. | Percentage deviation | Liver attachment min size (nm) $d_{a,apoE}$ min | Liver attachment shape (nm) $\sigma_{a,apoE}$ | ApoE uptake shape (nm) $\sigma_{u,apoE}$ | apoB reabsorption affinity (particles / fl*day) $k_{u,apoB}$ | Liver attachment max rate (particles / fl*day) $k_{a,apoE}$ max | LpL attachment max rate (particles / fl*day) $k_{lmax}$ |
|---|---|---|---|---|---|---|---|
| 1 | 5,5 | 22,6 | 198,8 | 33,1 | 0,94 | 149,1 | 378,8 |
| 2 | 1,5 | 24,2 | 34,1 | 866,5 | 0,74 | 27,1 | 101,6 |
| 3 | 4,1 | 25,1 | 37,9 | 443,4 | 0,45 | 14,6 | 98,8 |
| 4 | 12,9 | 24,7 | 30,3 | 166,7 | 0,56 | 3,6 | 167,8 |
| 5 | 3,1 | 23,5 | 58,5 | 37,8 | 0,37 | 26,6 | 17,2 |
| 6 | 1,7 | 24,9 | 33,0 | 92,8 | 0,48 | 9,9 | 67,5 |
| 7 | 8,9 | 24,6 | 148,0 | 1278,7 | 0,49 | 107,5 | 22,6 |
| 8 | 19,4 | 25,4 | 198,4 | 37,1 | 0,35 | 179,5 | 31,6 |
| 9 | 3,9 | 24,7 | 199,2 | 42,7 | 0,28 | 132,4 | 15,4 |
| 11 | 8,8 | 24,0 | 198,8 | 34,6 | 0,30 | 92,0 | 83,3 |
| 12 | 5,7 | 24,4 | 198,0 | 37,4 | 0,39 | 107,9 | 22,5 |
| 14 | 2,7 | 24,3 | 41,8 | 52,1 | 0,24 | 7,0 | 0,0 |
| 17 | 6,8 | 21,7 | 31,6 | 15083,8 | 0,38 | 3,5 | 55,4 |
| 18 | 16,7 | 13,3 | 36,6 | 48,7 | 0,36 | 3,9 | 61,7 |
| 19 | 5,4 | 23,4 | 32,7 | 37,9 | 0,33 | 5,4 | 48,1 |
| 20 | 5,4 | 19,4 | 199,4 | 38,9 | 0,27 | 32,9 | 16,0 |
| Averages | | | | | | | |
| A | | 24,4 | 104,2 | 333,2 | 0,52 | 72,2 | 100,1 |
| I | | 24,3 | 146,2 | 41,4 | 0,31 | 69,0 | 35,3 |
| B | | 19,4 | 75,1 | 3802,3 | 0,34 | 11,4 | 45,3 |
| Significance inter-group difference | | | | | | | |
| p | | 0,016 | 0,504 | 0,438 | 0,073 | 0,152 | 0,438 |

Table 1: The fitted model parameter values for 16 subjects from Packard et al. (2000, J. Lipid Res. 41:305-318) Only subjects with a data set corresponding to steady-state were selected. The patients were grouped by Packard et al. into three phenotype classes, according to their 'LDL peak size'. Class A had a peak size > 26 nm, class I between 25 and 26 nm and class B < 25 nm. Lower LDL peak size is thought to correspond to a higher risk for cardiovascular disease. The fitted model parameter average for each of these classes is given, and the significance of inter-group difference according to the nonparametric Kruskal-Wallis test.

**Prediction of LDL peak size shift**

The model could simulate detailed particle size profiles, although it is fitted to pools and fluxes of only four density categories (VLDL 1, VLDL 2, IDL and LDL). These detailed profiles were averaged for all patients in each phenotype class defined by Packard et al. In figure 4 these averaged profiles are shown. Although the A and I category profiles overlap, a shift towards lower LDL peak sizes was observed as the phenotype changes from A and I to B, corresponding to the peak size shift measured by Packard. This result points to the physiological realism of the model, since with no size data other than an estimation of the particle size ranges of each density category, the model still reproduced a LDL particle size shift.

The peak size shift was also visible in the model parameters. The Kruskal-Wallis test showed that the median of the lipolysis minimum size significantly decreased from group A and I to group B, with p =0,016. This shows that both the modeled size-concentration profiles and the model parameters qualitatively reproduced the LDL peak size shift between the groups.

**Process identification**

Table 2 shows the derived parameters that indicate the status of the various physiological processes. Next to the lipolysis minimum size, also the HL peak attachment rate has significantly different medians between the groups.
Table 3 shows the size-class specific indicator parameters with a significantly changed median between the groups. These include the VLDL1, VLDL2 and LDL average particle age, the IDL and LDL average particle size, the VLDL1 and VLDL2 average lipolysis attachment rate in general and specifically for HL, and the VLDL2 liver attachment rate.

A similar group-comparison analysis was done based on the flux parameters Packard reports in his paper (2000, J. Lipid Res. 41:305-318), and using the same patients as we did. In that case next to the pool sizes, only the transfer rate of VLDL1 to VLDL2 differs significantly between the groups. The model therefore seems to be able to indicate relevant differences in physiology between groups with a differing LDL peak size.

| Subj. | Liver Attachment min size (nm) | apoB reabsorption affinity (particles / fl*day) | HL peak attachment rate (part/fL*day) | HL attachment peak size (nm) | average apoE-mediated uptake attachment VLDL1 (part/fL*day) |
|---|---|---|---|---|---|
| 1 | 22,6 | 0,94 | 8,8 | 33,1 | 23,8 |
| 2 | 24,2 | 0,74 | 27,1 | 34,1 | 0,0 |
| 3 | 25,1 | 0,45 | 14,6 | 37,9 | 0,0 |
| 4 | 24,7 | 0,56 | 3,6 | 30,3 | 0,0 |
| 5 | 23,5 | 0,37 | 10,1 | 36,7 | 12,6 |
| 6 | 24,9 | 0,48 | 9,8 | 33,0 | 0,1 |
| 7 | 24,6 | 0,49 | 89,7 | 100,0 | 0,0 |
| 8 | 25,4 | 0,35 | 12,1 | 37,1 | 20,8 |
| 9 | 24,7 | 0,28 | 13,6 | 42,6 | 9,9 |
| 11 | 24,0 | 0,30 | 5,6 | 34,6 | 13,1 |
| 12 | 24,4 | 0,39 | 8,0 | 37,3 | 12,5 |
| 14 | 24,3 | 0,24 | 5,9 | 39,1 | 1,4 |
| 17 | 21,7 | 0,38 | 3,5 | 31,6 | 0,0 |
| 18 | 13,3 | 0,36 | 3,2 | 32,8 | 1,0 |
| 19 | 23,4 | 0,33 | 4,5 | 31,2 | 1,1 |
| 20 | 19,4 | 0,27 | 3,5 | 38,8 | 3,8 |
| Averages | | | | | |
| A | 24,4 | 0,52 | 21,0 | 42,7 | 7,5 |
| I | 24,3 | 0,31 | 6,5 | 37,0 | 9,0 |
| B | 19,4 | 0,34 | 3,7 | 33,6 | 1,5 |
| Significance inter-group difference | | | | | |

(continued)

| Subj. | Liver Attachment min size (nm) | apoB reabsorption affinity (particles / fl*day) | HL peak attachment rate (part/fL*day) | HL attachment peak size (nm) | average apoE-mediated uptake attachment VLDL1 (part/fL*day) |
|---|---|---|---|---|---|
| p | 0,016 | 0,073 | 0,007 | 0,284 | 0,360 |

Table 2: derived process indicator parameters for 16 subjects from packard et al. (2000, j. Lipid res. 41:305-318) only subjects with a data set corresponding to steady-state were selected. The patients were grouped by packard et al. Into three phenotype classes, according to their 'ldl peak size'. Class a had a peak size > 26 nm, class i between 25 and 26 nm and class b < 25 nm. Lower ldl peak size is thought to correspond to a higher risk for cardiovascular disease. The fitted model parameter average for each of these classes is given, and the significance of inter-group difference according to the nonparametric kruskal-wallis test. Significant differences between the groups are seen in the liver attachment minimum size, which shifts the ldl peak size in the model output. Also the hl peak attachment rate changes, indicating a change in hl activity.

| | Group means | | | Significance inter-group difference |
|---|---|---|---|---|
| | A | I | B | p-value |
| LDL average particle age (days) | 1,5 | 2,4 | 2,6 | 0,014 |
| VLDL2 average particle age (days) | 0,11 | 0,19 | 0,22 | 0,011 |
| VLDL1 average particle age (days) | 0,03 | 0,06 | 0,07 | 0,018 |
| LDL average particle size (nm) | 23,2 | 23,2 | 20,2 | 0,024 |
| IDL average particle size (nm) | 26,7 | 26,8 | 27,2 | 0,018 |
| Size-specific process indicator parameters | | | | |
| VLDL2 average lipolysis attachment rate (part/fL*day) | 12,7 | 6,2 | 4,4 | 0,005 |
| VLDL1 average lipolysis attachment rate (part/fL*day) | 30,8 | 13,1 | 11,5 | 0,019 |
| VLDL2 liver attachment 'rate' (part/fL*day) | 12,5 | 7,2 | 4,4 | 0,025 |
| VLDL2 HL lipolysis rate (part/fL*day) | 10,8 | 5,5 | 3,5 | 0,022 |
| VLDL1 HL lipolysis rate (part/fL*day) | 11,1 | 5,8 | 2,6 | 0,035 |
| Packard - from published process parameters | | | | |
| Transfer from VLDL1 to VLDL2 (pools/day) | 16,8 | 5,9 | 5,8 | 0.0144 |

Table 3: Derived size-specific indicator parameters that showed a significant difference (p<0.5%) between groups using the nonparametric Kruskal-Wallis test. The patients were grouped by Packard et al. (2000, J. Lipid Res. 41:305-318) into three phenotype classes, according to their 'LDL peak size'. Class A had a peak size > 26 nm, class I between 25 and 26 nm and class B < 25 nm. Lower LDL peak size is thought to correspond to a higher risk for cardiovascular disease. Testing data from the original publication showed a difference between groups in one process - transfer from VLDL 1 to VLDL 2. The current analysis showed five significantly different processes. It more sensitively indicated changes in the VLDL 2 region, as well as indicating a changed HL activity in the whole VLDL range.

**Prediction of polymorphism effects**

Figure 5 shows the model fit of patient 19 and simulated polymorphisms affecting ApoB-mediated reabsorption and LpL lipolysis affinity. The cholesterol and triglyceride concentrations in different size classes for the simulated ApoB-mediated reabsorption reduction show the expected hypercholesterolemia (Guerin, M., P. J. et al. 1995. Arterioscler Thromb Vasc Biol 15:1359-1368). The halved ApoB-related reabsorption affinity results in a 2.2-fold increase of the LDL-cholesterol concentration in the blood.

The modelled lipolysis affinity reduction also reproduces the expected hypertriglyceridemia (Okazaki, M., et al. 2005. Arterioscler Thromb Vasc Biol 25:578-584), although less severely than the hypercholesterolemia induced above. Downregulating the LpL lipolysis affinity (by 75%) results in a 2,2 fold increase of VLDL1-triglyceride concentration in the blood. The modelled genetic variants therefore qualitatively resemble the observed phenotype.

**Modelling a single measurement**

Figure 6 shows the rate parameter values of two patients estimated based on 6 subclasses as described above. The patients clearly differ in their individual parameters.

**Claims**

1. Population model for the analysis of blood lipoprotein physiology in a test subject comprising:

    a. a submodel for the production of blood lipoproteins;
    b. a submodel for the lipolysis of blood lipoproteins;
    c. a submodel for the reabsorption of blood lipoproteins; and
    d. a submodel relating blood lipoprotein particle size to biochemical composition, more specifically triglyceride content,

    thereby providing an analysis of the physiological processes underlying a steady state particle population distribution.

2. Deterministic population model according to claim 1, wherein each submodel is given as function, using the size of the lipoprotein particle as the independent variable.

3. Population model according to claim 1-2, wherein in the submodel for the lipolysis two models are contained, one for extra-hepatic tissue mediated lipolysis and one for hepatic lipolysis.

4. Population model according to claim 1-3, wherein the submodel for the reabsorption is able to distinguish between apoB and apoE mediated reabsorption.

5. Population model according to any of claims 1-4, further comprising one or more of the following submodels:

    a. a submodel relating blood lipoprotein particle size to total cholesterol content;
    b. a submodel relating blood lipoprotein particle size to free cholesterol content;
    c. a submodel relating blood lipoprotein particle size to cholesterol ester content;
    d. a submodel relating blood lipoprotein particle size to phospholipid content;
    e. a submodel relating blood lipoprotein particle size to total protein content.

6. Population model for the presence of blood lipoproteins in a test subject according to claim 1-5 wherein the total steady-state pool of lipoproteins $Q_{ss}$ in a diameter range $[d_a \ d_b]$ is given by:

$$Q_{ss}([d_a \quad d_b]) = \sum_{d_0} \sum_{d_i \in [d_a \ d_b]} Q_{ss}^{d_0}(d_i)$$

wherein $Q_{ss}^{d_0}(d_i)$ is the steady state pool of a cascade step at diameter $d_i$ resulting from the particle influx at $d_0$.

The steady-state pool in the cascade step $Q_{ss}^{d_0}(d_i)$ at each size $d$ resulting from the particle influx at $d_0$ is given by:

$$Q_{ss}^{d_0}(d_i) = J_{in}^{d_0}(d_i)\tau(d_i)$$

wherein $d$ is the particle diameter, $\tau$ signifies the residence time of a particle within a size class and $J_{in}^{d_0}(d_i)$ is the particle influx resulting from production if $d_i = d_0$ and from lipolysis otherwise.

7. Model according to claim 6, wherein the influx into the previous cascade step at particle diameter $d_{i-1}$ is defined as:

$$J_{in}(d_i) = J_{in}(d_{i-1}) \frac{k_l(d_{i-1}) + k_{l,liver}(d_{i-1})}{k_l(d_{i-1}) + k_{l,liver}(d_{i-1}) + k_{u,liver}(d_{i-1})}$$

where $k_{u,liver}$ is the particle reabsorption rate, $k_l$ is the extrahepatic lipolysis attachment rate, $k_{l,liver}$ is the hepatic lipolysis attachment rate and $d_{i-1}$ indicates the particle radius before the lipolysis step.

8. Model according to claim 6 or 7, in which the residence time of a particle within a size class is given by

$$\tau(d_i) = \frac{1}{k_{a,liver}(d_i) + k_l(d_i)}$$

where $k_{a,liver}$ is the rate at which particles are attached to the liver to either be taken up or lipolysed there, $k_l$ is the lipolysis attachment rate for extrahepatic lipolysis.

9. Model according to any of claims 6-8, wherein the production of blood lipoproteins is given by the

$$\text{equation } J_{in}^{d_0}(d_0) = \begin{cases} J_{in,LDL}\, f_{nor}\!\left(d_0, \overline{d_{LDL}}, \sigma_{LDL}\right) & for & d_0 \in [d_{LDL\min}, d_{LDL\max}] \\ J_{in,IDL}\, f_{nor}\!\left(d_0, \overline{d_{IDL}}, \sigma_{IDL}\right) & for & d_0 \in [d_{IDL\min}, d_{IDL\max}] \\ J_{in,VLDL2}\, f_{nor}\!\left(d_0, \overline{d_{VLDL2}}, \sigma_{VLDL2}\right) & for & d_0 \in [d_{VLDL2\min}, d_{VLDL2\max}] \\ J_{in,VLDL1}\, f_{lognor}\!\left(d_0, \mu_{VLDL1}, \sigma_{VLDL2}\right) & for & d_0 \in [d_{VLDL1\min}, d_{VLDL1\max}] \end{cases}$$

wherein $d_0$ stands for the particle diameter at production, $\overline{d}$ stands for the average diameter in a given class, $\sigma$ is the standard deviation of the distribution curve, subscripts indicate the class to which the diameter refers, and whether it is a minimum or maximum value for that class, $f_{nor}$ is the corrected normal probability density function, and $f_{lognor}$ is the corrected lognormal probability density function as follows:

$$f_{nor}(d, \overline{d}, \sigma) = \begin{cases} \dfrac{normpdf\!\left(d, \overline{d}, \sigma\right)}{normcdf\!\left(d_{\max}, \overline{d}, \sigma\right) - normcdf\!\left(d_{\min}, \overline{d}, \sigma\right)} \\ 0 \end{cases}$$

for $d > d_{\min}$ and $d < d_{\max}$ otherwise and

$$f_{lognor}(d, \mu, \sigma_{ln}) = \begin{cases} \dfrac{\log normpdf\!\left(d - d_{\min}, \mu, \sigma_{ln}\right)}{\log normcdf\!\left(d_{\max} - d_{\min}, \mu, \sigma_{ln}\right)} \\ 0 \end{cases}$$

for $d > d_{\min}$ and $d < d_{\max}$ otherwise
wherein $normpdf(d, \overline{d}, \sigma)$ stands for a normal probability density function with parameters $\overline{d}$ and $\sigma$ evaluated at $d$,

while *lognormpdf(d,μ,σ<sub>ln</sub>)* stands for a lognormal probability density function with parameters $\mu$ and $\sigma_{ln}$, evaluated at *d*.

**10.** Model according to any of claims 6-9, wherein the lipolysis attachment rate in extra-hepatic tissue is given by the formula

$$k_{l,eht}(d) = \left\{ \begin{array}{l} k_{l\max}\left(1 - \exp\dfrac{-(d - d_{l\min})^2}{2\sigma_l^2}\right) \\ 0 \end{array} \right.$$

*for $d \geq d_{l\min}$ otherwise*
wherein *d* is the particle diameter, $d_{l\min}$ is the minimum size at which lipolysis occurs, $k_{l\max}$ is the maximum lipolysis attachment rate and $\sigma_l$ is a shape parameter.

**11.** Model according to any of claims 6-10, wherein the lipolysis rate in the liver $k_{l,liver}$ is given by the formula

$$k_{l,liver}(d) = k_{a,liver}(d) - k_{u,liver}(d)$$

wherein the liver attachment rate $k_{a,liver}$ is given either by

$$k_{a,liver}(d) = \left\{ \begin{array}{l} k_{a,apoE\max}\left(\dfrac{(d - d_{a,apoE\min})\exp\dfrac{-(d - d_{a,apoE\min})^2}{2(\sigma_{a,apoE} - d_{a,apoE\min})^2}}{(\sigma_{a,apoE} - d_{a,apoE\min})\exp\dfrac{-(\sigma_{a,apoE} - d_{a,apoE\min})^2}{2(\sigma_{a,apoE} - d_{a,apoE\min})^2}}\right) + k_{a,apoB} \\ k_{a,apoB} \end{array} \right.$$

*for $d \geq d_{a,apoE\min}$ otherwise* or by

$$k_{a,liver}(d) = \left\{ \begin{array}{l} k_{a,apoE\max}\left(\dfrac{f_{weibullpdf}(d - d_{a,apoE\min}, A_{a,apoE\min}, B_{a,apoE\min})}{f_{weibullpdf\max}}\right) + k_{a,apoB} \\ k_{a,apoB} \end{array} \right.$$

*for $d \geq d_{a,apoE\min}$ otherwise* and the liver uptake rate $k_{u,liver}$ is given by

$$k_{u,liver}(d) = \left\{ \begin{array}{l} (k_{a,liver} - k_{a,apoB})\left(1 - \exp\dfrac{-(d - d_{a,apoE\min})^2}{2\sigma_{u,liver}^2}\right) + k_{a,apoB} \\ k_{a,apoB} \end{array} \right.$$

*for $d \geq d_{a,apoE\min}$ otherwise*
wherein $k_{a,apoEmax}$ is the maximum liver uptake rate due to apoE-mediated reabsorption, $k_{a,apoB}$ is the liver uptake rate due to apoB-mediated reabsorption, *d* is the particle diameter, $d_{a,apoEmin}$ is the minimum particle diameter at which liver lipolysis takes place and $\sigma_{a,apoE}$ and $\sigma_{u,liver}$ are shape parameters and where $f_{weibullpdf}(d,A,B)$ is a weibull probability density function evaluated at $d$ with shape parameters A and B, and $f_{weibullpdfmax}$ is the maximum function value attained by this weibull function on the lipoprotein size range from 0 to 200 nm.

12. Method to determine individual parameters of each submodel using data obtained from a blood sample in a subject comprising:

    a. taking a blood sample from said subject;
    b. providing a data set from said sample comprising either the number of blood lipoprotein particles in a size class or the chemical composition of said particles, wherein at least 6 size classes are provided;
    c. feeding said data to a model as claimed in claims 1-11;
    d. finding parameters for the submodels defined in claim 1-11 such that the resulting calculated total steady-state pool of lipoproteins $Q_{ss}$ for every diameter $d$ is in agreement with said dataset.

13. Method according to claim 12 wherein additionally from said sample or said subject one or more data are provided, including but not limited to data from the group consisting of the ApoC3 content, the ApoA5 content, insulin sensitivity indexes, the sialic acid content, the lipoprotein lipase activity, the hepatic lipase activity, the content of C-reactive protein, the content of adiponectin, gene expression data in blood cells, relevant single nucleotide polymorphisms and copy number variations.

14. Method to monitor the development of disease or the effect of a therapy in a patient by performing the method of claim 12 or 13.

15. Method according to any of claims 12-14, wherein said disease is selected from the group of lipid metabolism disorders, including but not limited to hyper-and hypocholesterolemia, hypertriglyceridemia and hyperlipoproteinemia types I, IIa, IIb, III, IV and V.

16. Use the method of any of claims 12-15 to choose a patient specific therapeutic intervention, directed at one or more processes that are described by one or more sub models relating to composition, production lipolysis and reabsorption of lipoproteins.

Figure 1.

Figure 2

Figure 3

Figure 4

Figure 5

Figure 6

**Europäisches Patentamt**
**European Patent Office**
**Office européen des brevets**

## PARTIAL EUROPEAN SEARCH REPORT

which under Rule 63 of the European Patent Convention shall be considered, for the purposes of subsequent proceedings, as the European search report

Application Number

EP 08 15 7988

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARTIN ADIELS: "PhD thesis: Compartmental Models of Lipoprotein Kinetics" 1 January 2004 (2004-01-01), CHALMERS UNIVERSITY OF TECHNOLOGY AND GÖTEBORG UNIVERSITY , GÖTEBORG , XP009108560 whole doc; in particular table 1.1 and fig.'s 1.1, 1.2, 1.4 and 2.3. * the whole document * ----- | 12-16 | INV. G06F19/00 G01N33/92 |
| A | WO 93/03450 A (UNIV NORTH CAROLINA [US]) 18 February 1993 (1993-02-18) * the whole document * ----- | 12-16 | |
| A | PACKARD C J ET AL: "Apolipoprotein B metabolism and the distribution of VLDL and LDL subfractions" JOURNAL OF LIPID RESEARCH, vol. 41, no. 2, February 2000 (2000-02), pages 305-317, XP002503621 ISSN: 0022-2275 * the whole document * ----- -/-- | 12-16 | |
| | | | TECHNICAL FIELDS SEARCHED (IPC) G06F G01N |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 13 November 2008 | Lüdemann, Susanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

**Europäisches Patentamt**
**European Patent Office**
**Office européen des brevets**

## PARTIAL EUROPEAN SEARCH REPORT

Application Number

EP 08 15 7988

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| A | ZHENG CHUNYU ET AL: "Rapid turnover of apolipoprotein C-III-containing triglyceride-rich lipoproteins contributing to the formation of LDL subfractions" JOURNAL OF LIPID RESEARCH, vol. 48, no. 5, May 2007 (2007-05), pages 1190-1203, XP002503622 ISSN: 0022-2275 * the whole document *<br><br>----- | 12-16 | |
| A | AUSTIN M A ET AL: "ATHEROGENIC LIPOPROTEIN PHENOTYPE A PROPOSED GENETIC MARKER FOR CORONARY HEART DISEASE RISK" CIRCULATION, vol. 82, no. 2, 1990, pages 495-506, XP002503623 ISSN: 0009-7322 * the whole document *<br><br>----- | 12-16 | **TECHNICAL FIELDS SEARCHED (IPC)** |

EPO FORM 1503 03.82 (P04C10)

Europäisches
Patentamt
European
Patent Office
Office européen
des brevets

**INCOMPLETE SEARCH
SHEET C**

Although claims  12-16    are directed to a diagnostic or therapeutic
methods practised on the human/animal body (Article 53(c) EPC), the
search has been carried out and based on the alleged effects of the
compound/composition.

        -----

Claim(s) not searched:
        1-11

Reason for the limitation of the search (non-patentable invention(s)):

Article 52 (2)(a) EPC - Mathematical method and model

**ANNEX TO THE EUROPEAN SEARCH REPORT
ON EUROPEAN PATENT APPLICATION NO.**

EP 08 15 7988

This annex lists the patent family members relating to the patent documents cited in the above-mentioned European search report.
The members are as contained in the European Patent Office EDP file on
The European Patent Office is in no way liable for these particulars which are merely given for the purpose of information.

13-11-2008

| Patent document cited in search report | Publication date | Patent family member(s) | Publication date |
|---|---|---|---|
| WO 9303450 A | 18-02-1993 | AU 2384292 A<br>US 5343389 A | 02-03-1993<br>30-08-1994 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5343389 A

### Non-patent literature cited in the description

- **Austin, M.A. et al.** *Circulation,* 1990, vol. 82, 495-506
- **Alaupovic, P.** *Meth. Enzymol.,* 1996, vol. 263, 32-60
- **Walldius, G. et al.** *J. Intern. Med.,* 2006, vol. 259, 259-266
- **Fisher, W.R. et al.** *J. Lipid Res.,* 1980, vol. 21, 760-774
- **Fisher, W.R. et al.** *J. Lipid Res.,* 1991, vol. 32, 1823-1836
- **Packard, C.J. et al.** *J. Lipid Res.,* 1995, vol. 36, 172-187
- **Maistrom, R. et al.** *Arterioscler. Thromb. Vasc. Biol.,* 1997, vol. 17, 1454-1464
- **Harris, W.S. et al.** *J. Lipid Res.,* 1990, vol. 31, 1549-1558
- **Barrett, P.H. et al.** *J. Lipid Res.,* 1991, vol. 32, 743-762
- **Patterson, B.W. et al.** *J. Lipid Res.,* 2002, vol. 43, 223-233
- **Campos, H. et al.** *J. Lipid Res.,* 2001, vol. 42, 1239-1249
- **Cohn, J.S. et al.** *J. Clin. Endocrinol. Metab.,* 2004, vol. 89, 3949-3955
- **Zheng, C. et al.** *J. Lipid Res.,* vol. 48, 1190-1203
- **Tuzikov et al.** *Voprosy Meditsinskoj Khimii,* 2002, vol. 48, 90-91
- **Packard et al.** *J. Lipid Res.,* 2000, vol. 41, 305-318
- **Adiels et al.** *J. Lipid Res.,* 2005, vol. 46, 58-67
- **Lichtenstein, L. et al.** *Arterioscler. Thromb. Vasc. Biol.,* 2007, vol. 27, 2420-2427
- **Demant, T. et al.** *J. Lipid Res.,* 1993, vol. 34, 147-156
- **de Beer, F. et al.** *Arterioscler. Thromb. Vasc. Biol.,* 1999, vol. 19, 633-637
- **Adiels, M.** *PhD thesis,* 2004
- **Demant, T. et al.** *J. Lipid Res.,* 1988, vol. 29, 1603-1611
- **Out, R. et al.** *J. Biol. Chem.,* 2004, vol. 279, 18401-18406
- **MacArthur, J. et al.** *J. Clin. Invest.,* 2007, vol. 117, 153-164
- **Packard et al.** *J. Lipid Res.,* 2000, vol. 41, 305-318
- *J. Lipid Res.,* 2000, vol. 41, 305-318
- *j. Lipid res.,* 2000, vol. 41, 305-318
- **Guerin, M., P. J. et al.** *Arterioscler Thromb Vasc Biol,* 1995, vol. 15, 1359-1368
- **Okazaki, M. et al.** *Arterioscler Thromb Vasc Biol,* 2005, vol. 25, 578-584